# EUROPEAN PATENT APPLICATION

(11) **EP 1 167 382 A1**
(43) Date of publication of application: **02.01.2002**
(21) Application number: 00911385.3
(22) Date of filing: 27.03.2000
(51) Int. Cl.: C07K 7/08, C07K 16/28, A61K 31/00, A61K 38/00

(54) **INFLUENZA VIRUS HEMAGGLUTININ-BINDING PEPTIDES**

(30) Priority: 31.03.1999 JP 9196299
(71) Applicant: OTSUKA PHARMACEUTICAL CO., LTD., Chiyoda-ku Tokyo 101-8535 (JP)
(72) Inventor: SATO, Toshinori, Fujisawa-shi, Kanagawa 251-0004 (JP); ISHIKAWA, Dai, Tokushima-shi, Tokushima 770-0002 (JP); TANAKA, Michinori, Itano-gun, Tokushima 771-1265 (JP); OGINO, Koichi, Naruto-shi, Tokushima 772-0003 (JP); TAKI, Takao, Itano-gun, Tokushima 771-0204 (JP)
(74) Representative: HOFFMANN - EITLE
(86) International application number: JP0001867
(87) International publication number: WO0059932

(57) **Abstract**

In accordance with this invention there is provided an influenza virus hemagglutinin-binding peptide having any of the amino acid sequences defined under SEQ ID NO:1 to NO:11. This peptide binds specifically to the hemagglutinin associated with the first step of influenza virus infection to prevent binding of the virus to the host receptor and, as such, finds application as a prophylactic drug for influenza virus infection or a therapeutic drug for influenza.

## Description

### TECHNICAL FIELD

The present invention relates to peptides having specific binding affinities for influenza viruses. More particularly, the invention relates to a peptide specifically binding to the hemagglutinin on the influenza virus envelope.

Infection of a host with an influenza virus is established as the virus binds to the host cell receptor via the hemagglutinin anchored in the virion envelope. The peptide of the invention binds itself to the hemagglutinin to preclude binding of the influenza virus to the host cell receptor and thereby contribute significantly to prevention of an infection with the influenza virus.

The present invention, therefore, is directed to use of said peptide as an inhibitor of the hemagglutinin-mediated binding of an influenza virus to the host receptor and to use thereof as an antiinfluenza drug.

### BACKGROUND ART

Present in the virion envelope of an influenza virus are two different spike glycoproteins, namely hemagglutinin (HA) and neuraminidase (NA, sialidase), both are playing important roles in the establishment of an infection with the virus and the release (emergence) of the virus from the host cell. The hemagglutinin, the former, recognizes as its receptor and binds specifically to the sialic acid-containing sugar chain ubiquitous on the cell membranes of hosts, namely man and other animals (mammals, birds, reptiles, fishes, amphibians, etc.) to induce endocytosis of the influenza virus into the host cell. On the other hand, neuraminidase, the latter, is a receptor-degrading enzyme which plays the role of cleaving the sialic acid residue of its own or on the host cell membrane to allow the emergence or release of virus particles from the host cell.

Influenza viruses are classified, according to the antigenicity of viral neucleoprotein and matrix protein present internally in the virion envelope, into type A, type B or type C. Influenza A viruses, in particular, are responsible for the repeated epidemics on a worldwide scale, which are due to their inter-subtype variations arising from the antigenic drifts by DNA hybridization, point mutation and other causes.

As mentioned above, the hemagglutinin associated with the first step of an influenza virus infection occurs in various subtypes according to the diversity of amino acid sequences of the antigenic determinant regions (A∼E) which are highly mutatable. The inter-subtype variation in the amino acid sequence of hemagglutinin is as large as 25∼75% but the so-called receptor binding pocket region where the host cell receptor is bound is comparatively mutation-free and its three-dimensional structure is well conserved (Y. Suzuki, Prog. Lipid. Res., 33, 429 (1994)).

Heretofore, with a view to preventing influenza virus infection, studies have been undertaken for the development of a vaccine which would specifically bind the hemagglutinin contributory to the establishment of an infection and thereby inhibit its function.

For example, by the technique of screening for sugars specifically binding to the receptor binding site of hemagglutinin using various sugar analogs developed on the rationale that the hemagglutinin recognizes and binds itself to the sialic acid-containing sugar chain of the host receptor, a variety of hemagglutinin-binding sugar analogs have so far been prepared (R. Roy, et al., J. Chem. Soc., Chem. Commun., 1869 (1993); M. Mammen, et al., J. Med. Chem., 38, 4179 (1995); T. Sato, et al., Chem. Lett., 145 (1999); M. Itzstein, et al., Nature, 363, 418 (1993)).

Aside from the above, there is a technique comprising constructing an antibody (anti-ideotype antibody) to the antigenic type (ideotype) of the antigen-binding site of a monoclonal antibody to the hemagglutinin receptor sugar chain. The principle involved here is that of constructing the amino acid sequence of the supervariable region of an antiideotype antibody which, in spatial configuration, resembles the three-dimentional structure of the sialic acid and sialosugar chain functioning as the hemagglutinin receptor as a substitute for said three-dimensional structure and let it mimic the hemagglutinin receptor of the host cell [Suzuki Yasuo, Viral Infection and Sugar Chain, Nikkei Science supplemental issue "Sugar Chain and Cell", 89∼101, October 1994].

However, these are invariably specific to various subtypes of hemagglutinin and, moreover, their binding constants are not high.

Therefore, the advent of a broad-spectrum vaccine acting on influenza viruses in general, regardless of subtypes, is awaited.

Thus, if there could be provided a substance capable of recognizing and binding to the hemagglutinin of influenza virus specifically, that is to say a substance capable of acting as a receptor of hemagglutinin in lieu of the host cell receptor, it should be able to competitively suppress or inhibit the binding of an influenza virus to the host receptor through the hemagglutinin, thus making it possible to prevent influenza virus infection.

Moreover, if the above substance is one which specifically recognizes and binds the "receptor binding pocket" of hemagglutinin which is well conserved among subtypes of influenza virus, it is expected that the substance will be able to prevent infection with influenza viruses in general regardless of subtypes.

The present invention has for its object providing an influenza virus hemagglutinin-binding peptide having such efficacy.

### BRIEF DESCRIPTION OF THE DRAWINGS

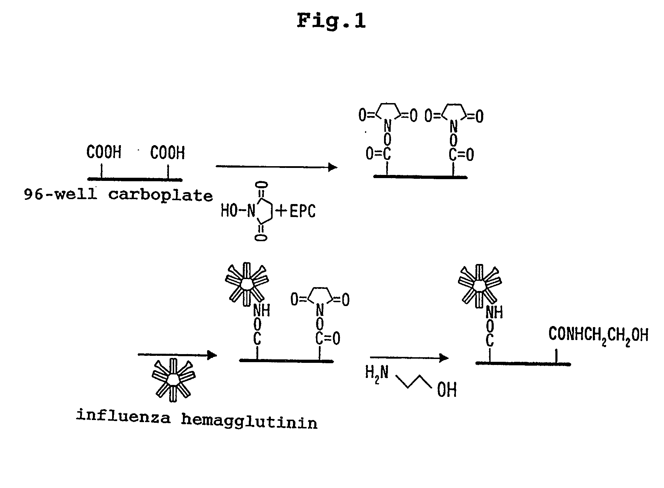

Fig. 1 is a schematic diagram showing a step of immobilizing influenza virus hemagglutinin on a microtiter plate.

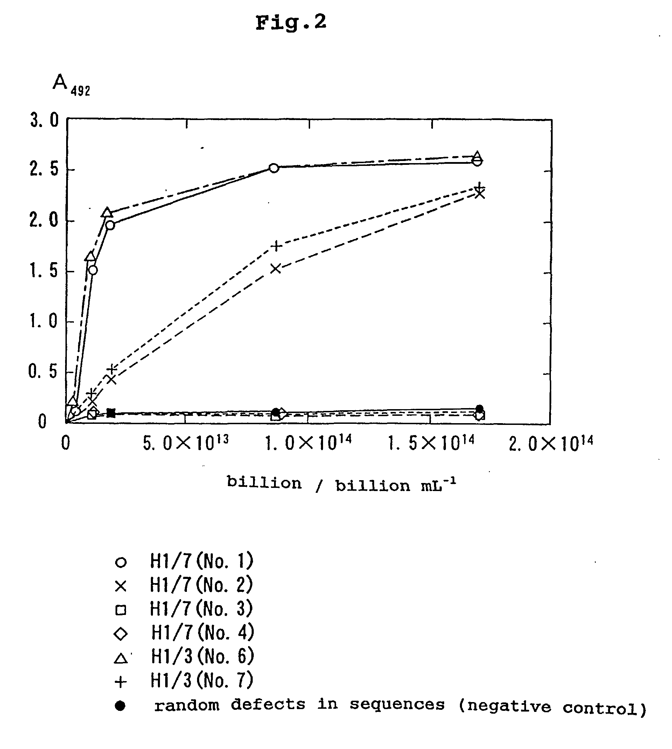

Fig. 2 is a diagram showing the results of a study of the hemagglutinin H1-binding affinity of the H1 phages (H1/7 phage and H1/3 phage) obtained by panning with hemagglutinin H1 as the target.

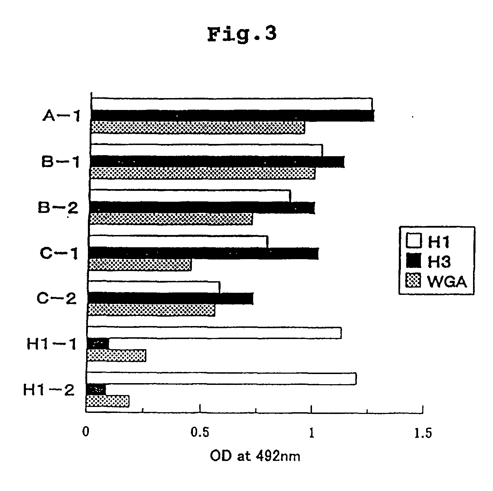

Fig. 3 is a diagram showing the results of a study of the binding affinities of the phage clones obtained in Example 1 and Example 3 ("A-1", "B-1", "B-2", "C-1", "C-2", "H-1-1" and "H1-2") for influenza hemagglutinins H1 and H3 and wheat germ agglutinin (WGA) in Example 4.

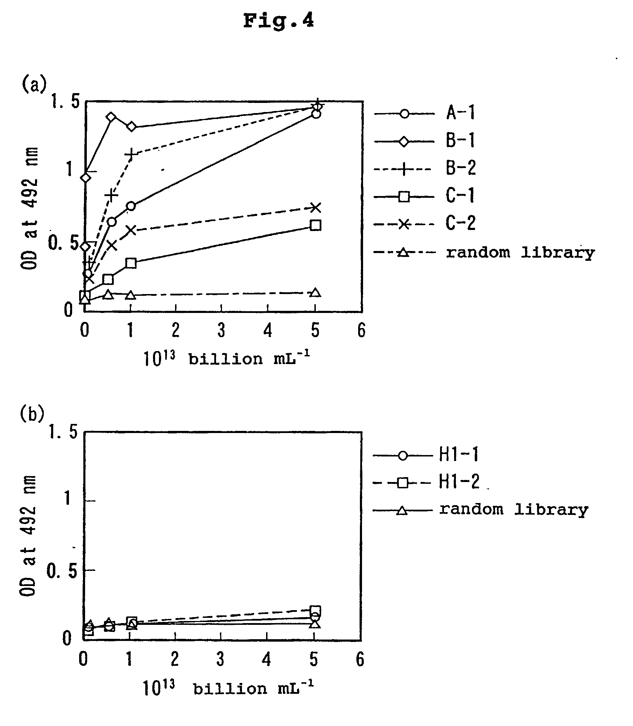

Fig. 4 is a diagram showing the results of a study of the binding affinities of the phage clones obtained in Example 1 and Example 3 ("A-1", "B-1", "B-2", "C-1" and "C-2" (all shown in Fig. 4(a)), "H1-1" and "H1-2" (both shown in Fig. 4 (b)) for anti-GM3 antibody in Example 5. In the drawing, "random library" (indicated by -△- on the graph) means a phage set expressing random peptides prior to panning.

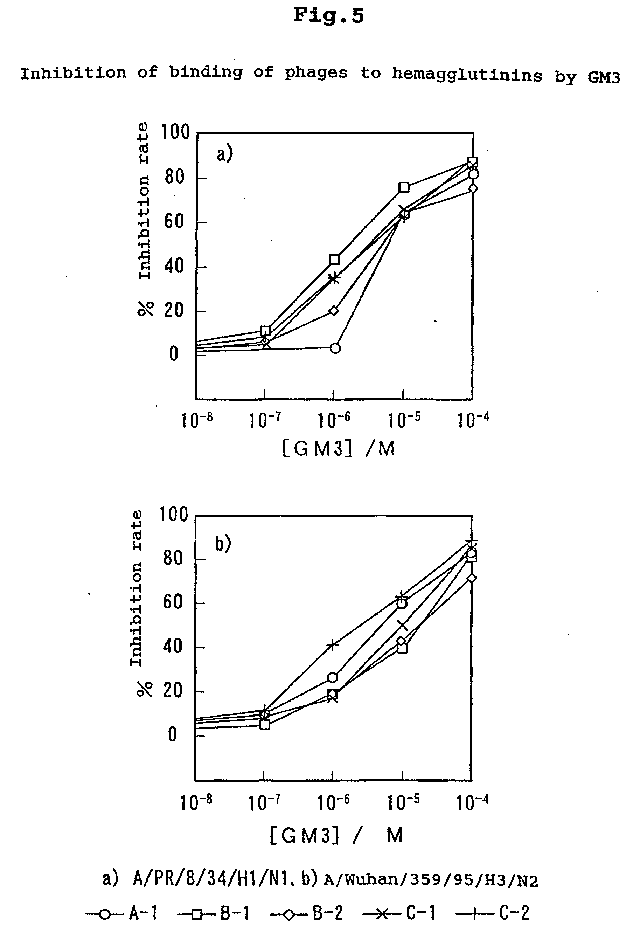

Fig. 5 is a diagram showing the results of a study of the inhibitory effect of ganglioside GM3 on the binding of phage clones ("A-1", "B-1", "B-2", "C-1" and "C-2") to hemagglutinin in Example 7. Fig. 5(a) represents the results obtained when H1 subtype (A/PR/8/34 (H1N1)) was used as the hemagglutinin and (b) represents the results obtained when H3 subtype (A/Wuhan/359/95 (H3N2)) was used as the hemagglutinin.

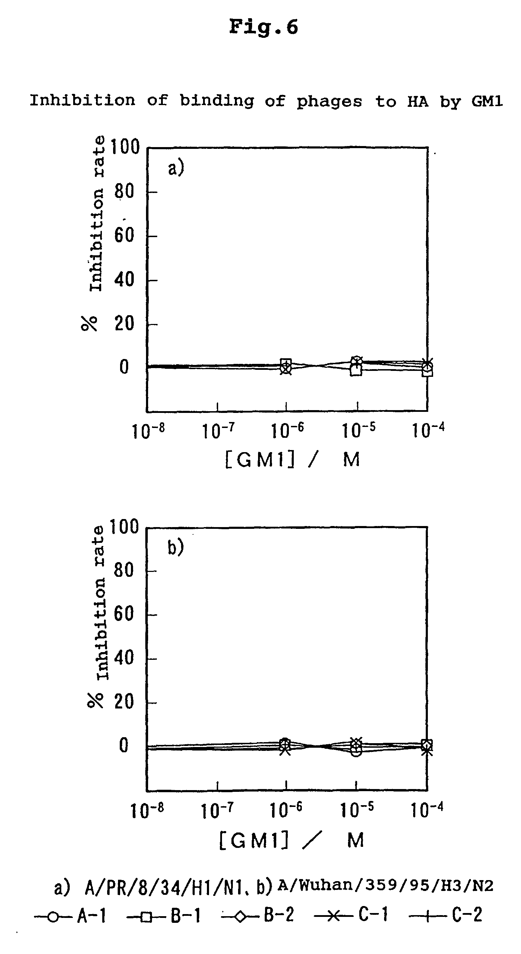

Fig. 6 is a diagram showing the results of a study of the inhibitory effect of ganglioside GM1 on the binding of phage clones ("A-1", "B-1", "B-2", "C-1" and "C-2") to hemagglutinin in Example 7. Fig. 6(a) represents the results obtained when H1 subtype (A/PR/8/34 (H1N1)) was used as the hemagglutinin and b) represents the results obtained when H3 subtype (A/Wuhan/359/95 (H3N2)) was used as the hemagglutinin.

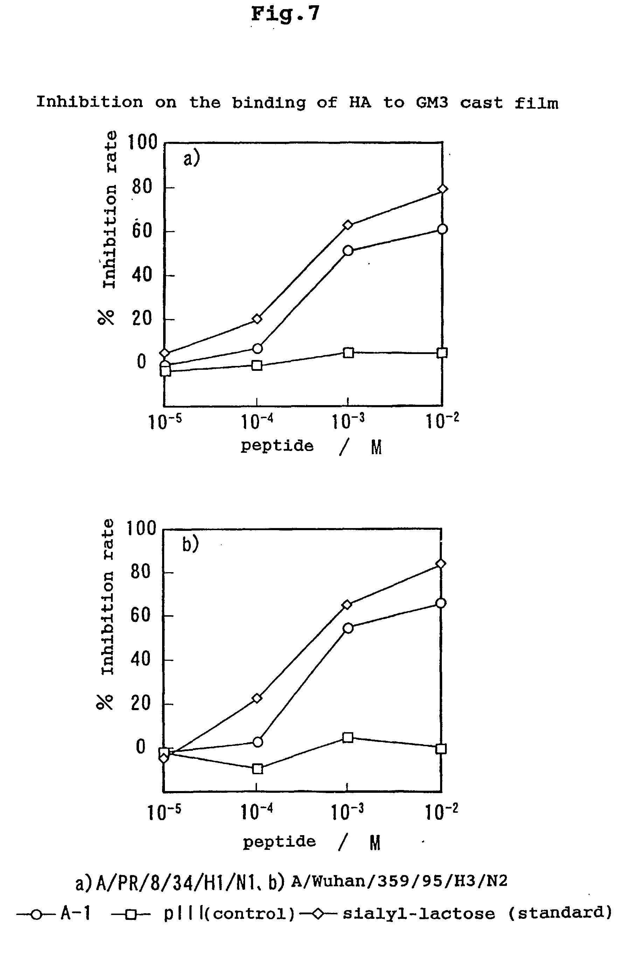

Fig. 7 is a diagram showing the results of a study of the inhibitory effect of phage clone ("A-1")-expressed peptide (synthetic peptide) on the binding of hemagglutinin to ganglioside GM3 (GM3 cast film) (indicated by -○- on the graph) in Example 11. Fig. 7(a) represents the results obtained when H1 subtype (A/PR/8/34 (H1N1)) was used as the hemagglutinin and b) represents the results obtained when H3 subtype (A/Wuhan/359/95 (H3N2)) was used as the hemagglutinin. In the drawing, -□- represents the results obtained with the N-terminal 15-residue peptide of the peptide-inserted pIII protein (control) and - 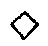 - represents the results obtained with sialyl-lactose (standard).

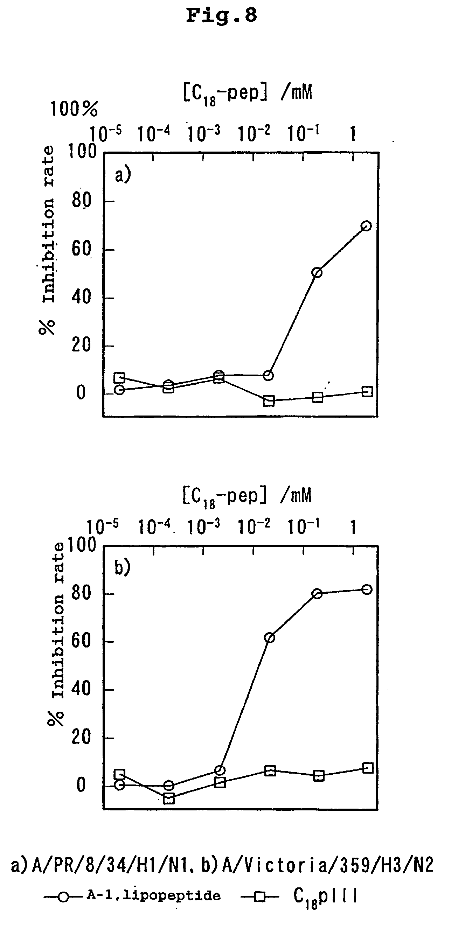

Fig. 8 is a diagram showing the results of a study of the inhibitory activity of the lipopeptide (C₁₈ A-1 lipopeptide) prepared in Example 9 against influenza virus infection of cells in Example 12. Fig. 8(a) represents the results obtained when H1 subtype (A/PR/8/34 (H1M1)) was used as the influenza virus and b) represents the results obtained when H3 subtype (A/Wuhan/359/95 (H3N2)) was used as the influenza virus. In the drawing, -○- represents the results with C₁₈ A-1 lipopeptide and -□- represents the results with a stearic acid derivative of the pIII protein N-terminal 15-residue peptide.

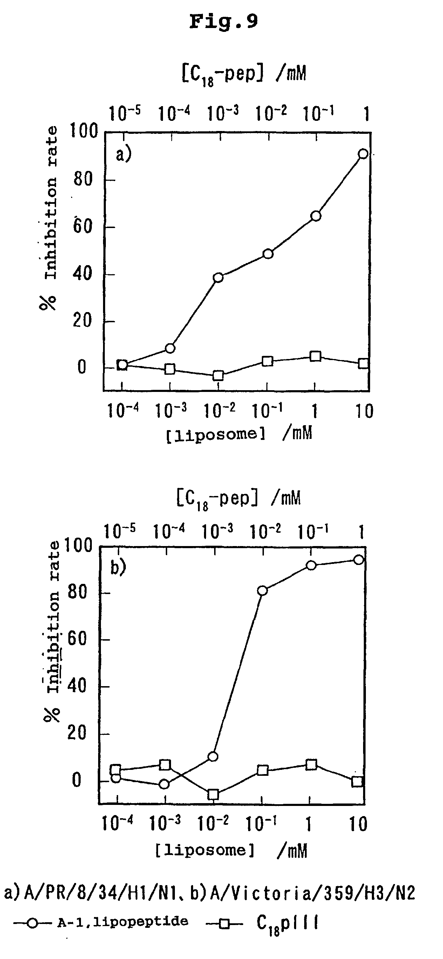

Fig. 9 is a diagram showing the results of a study of the inhibitory activity of the lipopeptide (C₁₈ A-1 lipopeptide)-modified liposome prepared in Example 9 against the influenza virus infection of cells in Example 12; a) represents the results obtained when H1 subtype (A/PR/8/34 (H1N1)) was used as the influenza virus and b) represents the results obtained when H3 subtype (A/Wuhan/359/95 (H3N2)) was used as the influenza virus. In the drawing, -○ - represents the results with C₁₈ A-1 lipopeptide and -□- represents the results with the stearic acid derivative of pIII protein N-terminal 15-residue peptide.

### DISCLOSURE OF INVENTION

The inventors of the present invention did an intensive research to accomplish the above object and confirmed that a peptide specifically recognizing and binding influenza virus hemagglutinin can be selectively obtained by utilizing the phage display technique.

The present invention has been developed on the basis of the above finding.

The present invention, therefore, is directed to the influenza virus hemagglutinin-binding peptides defined in the following paragraphs 1∼6:
1. An influenza virus hemagglutinin-binding peptide meeting whichever of the following definitions, (a) or (b).
   (a) A peptide having an amino acid sequence selected from among the amino acid sequences defined under SEQ ID NOs:1 ∼ 11.
   (b) A peptide having a modified amino acid sequence derived from the amino acid sequence according to the above paragraph (a) by the substitution, deletion or addition of 1 or a plurality of amino acids and having influenza virus hemagglutinin-binding activity.
2. An influenza virus hemagglutinin-binding peptide having the amino acid sequence defined under SEQ ID NO:1 or 6.
3. An influenza virus hemagglutinin-binding peptide having an amino acid sequence defined under any of SEQ ID NOs:7 ∼ 11.
4. The influenza virus hemagglutinin-binding peptide defined in any of the above paragraphs 1 through 3 wherein the influenza virus is a Type A virus.
5. The influenza virus hemagglutinin-binding peptide defined in any of the above paragraphs 1 through 3 wherein the influenza virus is an H1 subtype virus.
6. The influenza virus-hemagglutinin-binding peptide defined in the above paragraph 1 or 3 wherein the influenza virus is an H3 subtype virus.

These peptides may be alkylated or modified with lipids (phospholipids) to give lipopeptides. Therefore, the peptide of the invention includes such lipopeptides.

Furthermore, the present invention is directed to a liposome containing such a lipopeptide.

In addition, the present invention is directed to an influenza virus hemagglutinin binding inhibitor or antagonist comprising at least one species of the above-described influenza virus hemagglutinin-binding peptide as an active ingredient.

The present invention is further directed to the following pharmaceutical compositions which are of use as antiinfluenza drugs, specifically as prophylactic or therapeutic drugs for influenza.
(A) A pharmaceutical composition comprising at least one of the above-described influenza virus hemagglutinin-binding peptides as an active ingredient and a pharmaceutically acceptable carrier.
(B) The pharmaceutical composition defined above wherein the active ingredient influenza virus hemagglutinin-binding peptide is contained in the form of a lipopeptide.
(C) The pharmaceutical composition defined above wherein the active ingredient influenza virus hemagglutinin-binding peptide is present in the form of a liposome modifier.
(D) Any of the above pharmaceutical compositions the target influenza virus of which is a Type A virus.
(E) Any of the above pharmaceutical compositions the target influenza virus of which is a Type A H1 subtype virus.
(F) Any of the above pharmaceutical compositions the target influenza virus of which is a Type A H3 subtype virus.
(G) Any of the above pharmaceutical compositions for use as a prophylactic or therapeutic drug for influenza.

The present invention is further directed to a method for prophylaxis or therapy of influenza which comprises administering any of the above-mentioned pharmaceutical compositions (A)∼(G) to a recipient individual. The recipient includes those individuals susceptible to infection with influenza viruses [man and other animals (inclusive of mammals, birds, reptiles, fishes and amphibians) and those infected with an influenza virus. The preferred are individuals in whom the influenza virus involved is a type A virus and the more preferred are those in which the virus involved belongs to type A H1 subtype or H3 subtype.

The present invention is further directed to use of the above-described influenza virus hemagglutinin-binding peptide or peptide-modified liposome as said antiinfluenza drug (a prophylactic or therapeutic drug for influenza) and further to use of said influenza virus hemagglutinin-binding peptide or peptide-modified liposome in the production of a pharmaceutical composition for use as said antiinfluenza drug (prophylactic or therapeutic drug for influenza).

The representation of amino acids, peptides, nucleotide sequences, nucleic acids, etc. by abbreviations in this specification is in accordance with the rules of IUPAC, IUB, Guidelines for Drafting Specifications inclusive of Nucleotide Sequences or Amino Acid Sequences (ed. by the Patent Office of Japan), and the conventions in the art.

As specific examples of the influenza virus hemagglutinin-binding peptide of the invention, the peptides having the amino acid sequences defined under SEQ ID NOs:1∼11 as obtainable by the procedures shown in Examples which appear hereinafter can be mentioned.

The method of screening for a hemagglutinin-binding peptide (hereinafter referred to also as HA-binding peptide) of the invention, the characterization of the peptide obtained thereby, and the hemagglutinin-binding affinity of the peptide are now explained.

In the screening for the HA-binding peptide of the invention and the identification or characterization thereof, a molecular library screening technique can be employed, and as the library, a phage display library, among others, can be used with advantage.

As such a library, a commercial library can be utilized. With the random peptide display phages in the library, a peptide binding to a specific target molecule or cell can be selected using said molecule or cell in vitro and, moreover, the peptide so selected can be expressed. Therefore, the library is useful for the selection and identification of a peptide specifically binding to a target molecule or cell. The screening technique using this phage display library is known as the phage display method and has heretofore been used for the selection and identification of ligands specifically binding to various cell surface receptors or of various antibodies. Regarding the method of constructing a phage display library and the method for in vitro screening, reference may be made to the reports of Scott and Smith (Scott, J. M. and Smith, G. P., Science, 249, 386-390 (1990); Smith, G. P. and Scott. J. K., Methods in Enzymology, 217, 228-257 (1993)). These reports are incorporated by reference in this specification.

The HA-binding peptide of the invention can be acquired by carrying out the above in vitro screening for hemagglutinin-binding peptides by said phage display method. The following specific procedure may be used.

First, using a random peptide display phage constructed so as to express a peptide having a random amino acid sequence on the phage capsid by inserting a random DNA sequence into a known library, the display phage is reacted with the influenza virus hemagglutinin immobilized on the surface of a solid phase such as a microtiter plate and the phage which binds specifically to said hemagglutinin is recovered (biopanning).

The influenza virus hemagglutinin to be immobilized on a microtiter plate is not particularly restricted insofar as it is conservatively possessed of at least the "receptor-binding pocket" which binds to the host cell receptor. For example, it may be a very hemagglutinin or an influenza virus as such or even an influenza virus extract prepared by extracting the influenza virus with an organic solvent such as ether. The type of influenza virus is not particularly restricted, either, but, depending on the objective, may be any of types A, B and C viruses, viruses isolated from humans, viruses isolated from other mammals such as swine and equine species, and viruses of the avian origin. The preferred are type A viruses and viruses isolated from humans.

As mentioned above, the three-dimensional structure of the "receptor binding pocket" of a hemagglutinin is well conserved regardless of subtypes of the influenza virus. Therefore, the subtype of said influenza virus hemagglutinin to be immobilized on a microtiter plate is not restricted but the Type A H1 subtype and H3 subtype viruses isolated from humans in the most recent decade or so can be mentioned as preferred examples.

The phage binding specifically to the hemagglutinin can be recovered by permitting an HA receptor substance which competes with the host cell receptor in the binding to the hemagglutinin or an HA inhibitor or receptor antagonist capable of inhibiting the binding of the hemagglutinin to the host cell receptor to act upon the immobilized hemagglutinin. Thus, the phage bound specifically to the hemagglutinin immobilized on a microtiter plate can be eluted for recovery by reacting said HA receptor substance or HA inhibitor with the phage.

The HA receptor substance mentioned above is not particularly restricted insofar as it is capable of binding specifically to the hemagglutinin. In order to recover the phage bound specifically to the receptor-binding pocket of the hemagglutinin, it is recommendable to use an HA receptor substance capable of binding specifically to the receptor-binding pocket of the hemagglutinin. Such HA receptor substances are not restricted but ganglioside GM3, α (2→6) GM3, and sialyl-Lewis X, among others, can be mentioned.

The HA inhibitor or antagonist is not particularly restricted insofar as it is capable of inhibiting the binding of the hemagglutinin to the host cell receptor. Thus, for example, the sialic acid derivative 7-F-Neu5Ac2en, 2,7-dideoxy-7-fluoro-2,3-didehydro sialic acid, sialic acid dendrimer, and sialic acid-containing polymers can be mentioned.

Escherichia coli is infected with the phage thus obtained and cultured on a high production scale, followed by separation and purification to give a phage expressing a peptide binding specifically to the hemagglutinin, preferably a phage expressing a peptide binding specifically to the receptor-binding pocket of the hemagglutinin. The phage thus obtained is submitted to the panning procedure for screening for a phage specifically binding to the hemagglutinin of the influenza virus by the reaction with the immobilized hemagglutinin in the same manner as described above.

By repeating the above panning procedure a few times, preferably about 4∼6 times, a phage capable of expressing peptide binding specifically to the hemagglutinin, preferably a phage capable of expressing peptide binding specifically to the receptor binding pocket of the hemagglutinin can be selected.

Then, DNA is extracted from the selected phage and sequenced to identify the peptide expressed by the phage, that is to say the peptide binding specifically to the influenza virus hemagglutinin (HA-binding peptide), preferably the peptide binding specifically to the receptor binding pocket of said hemagglutinin.

The above DNA sequencing can be easily carried out by any of the methods known in the art, for example the dideoxy method [Proc. Natl. Acad. Sci. USA., 74, 5463-5467 (1977)] and the Maxam-Gilbert method [Method in Enzymology, 65, 499 (1980)]. The determination of nucleotide sequences can be easily made using a commercial sequencing kit as well.

As the phage library for the above phage display technique, any known phage library that has been generally used for the like purposes can be employed. Though this is not an exclusive choice, a random peptide display phage (filamentous phage) constructed by inserting a random DNA in the coat protein pIII gene of the phage so that a peptide having a random amino acid sequence of 15 residues may be expressed on the surface of the phage capsid can be mentioned as a preferred example [Title of speech: 2C103 "Selection of glycolipid-binding peptides by the phage display technique", The Third Symposium of Biotechnology Group of The Chemical Society of Japan (1998); JP Patent Application H11-000769].

The affinity (binding affinity) of the thus-identified HA-binding peptide for the hemagglutinin can be verified and evaluated by the same method as the above-described method of screening (panning) for a phage capable of expressing said HA-binding peptide except that said identified HA-binding peptide as the object of determination is used in lieu of the random peptide display phage.

As an embodiment of the invention, a protocol for selecting a phage expressing an HA-binding peptide by using an ether extract of A/PR/8/34 (H1N1), which is an influenza A virus (H1 subtype), as the immobilized influenza virus (hemagglutinin) and either an HA receptor substance or an HA inhibitor as the eluent and identifying the expressed peptide is described hereinafter in Example 1.

The peptide which can be identified by such an embodiment of the invention has any of the amino acid sequences defined under SEQ ID NOs:1∼6, and these peptides are invariably characterized in that they have binding affinities for the influenza virus hemagglutinin, particularly the receptor-binding pocket of the hemagglutinin. Furthermore, these peptides are characterized in that they have binding affinities for type A or human virus hemagglutinin among various influenza viruses. These peptides are selected by using hemagglutinin H1 subtype as the target and, therefore, characterized by their specific binding to this subtype. Particularly preferred examples of the peptide binding specifically to H1 subtype are those having the amino acid sequences defined under SEQ ID NO:1 and NO:5.

As a further embodiment of the invention, a protocol using an ether extract of the type A H1 subtype A/PR/8/34 (H1N1) and that of the H3 subtype A/Wuhan/359/95 (H3N2) as the immobilized influenza viruses (hemagglutinins) and various HA receptor substances (Sialyl-LewisX, α (2→6) GM3) as the eluents to select phages expressing a peptide which recognizes and binds both of these hemagglutinins and identifying the expressed peptide is described hereinafter in Example 3.

The peptide identified by this protocol according to the invention has one of the amino acid sequences defined under SEQ ID NO:7 through NO:11. These HA-binding peptides are characterized by the ability to bind both hemagglutinin subtypes H1 and H3. Since it is known that the H1 subtype and H3 subtype of hemagglutinin are 75% dissimilar in amino acid sequence, it appears that the above HA-binding peptides bind these subtypes by recognizing a highly homologous region of H1 and H3 subtypes of hemagglutinin.

Furthermore, among the above HA-binding peptides, the peptide corresponding to SEQ ID NO:7 in particular can be eluted and isolated with either of the two different eluents (sialyl-LewisX and α (2→6) GM3) and, therefore, the site of its binding to the hemagglutinin is the same as the site of binding of sialyl-LewisX and α (2→6) GM3 to the hemagglutinin and the mode of binding involved is also considered to be the same.

The peptide of the invention includes not only the above peptide having any of the amino acid sequences defined under SEQ ID NO:1 through NO:6 and the peptide having any of the amino acid sequences defined under SEQ ID NO:7 through NO:11 but also any and all peptides, inclusive of proteins, that have modified amino acid sequences derived from said amino acid sequences by substitution, deletion or addition of one or a plurality of amino acids and capable of binding to influenza virus hemagglutinins, particularly influenza A virus hemagglutinins.

Referring to the peptides represented by SEQ ID NO:1 through NO:6, the extent and amino acid positions of said "substitution, deletion or addition" are not particularly restricted insofar as the resulting mutant peptide or protein is equivalent to the peptide having any of the amino acid sequences defined under SEQ ID NO: 1 through NO:6 in the above-mentioned characteristic parameter, namely a binding affinity for an influenza virus hemagglutinin, particularly the receptor-binding pocket of the hemagglutinin. The preferred characteristic parameter may be a specific binding affinity for the H1 subtype of hemagglutinin.

Referring to the peptides defined under SEQ ID NO:7 through NO:11, the extent and amino acid positions of said "substitution, deletion or addition" are not particularly restricted insofar as the resulting mutant peptide or protein is equivalent to the peptide having any of the amino acid sequences defined under SEQ ID NO:7 through NO:11 in the above-mentioned characteristic parameter, namely a binding affinity for an influenza virus hemagglutinin, particularly the receptor-binding pocket of the hemagglutinin. The preferred equivalent peptide is a peptide which characteristically binds to both the H1 subtype and H3 subtype of hemagglutinin.

Such modification (mutation) of an amino acid sequence may take place through spontaneous mutation or posttranslational modification but can also be induced artificially. The present invention encompasses all modified or mutant peptides having the above characteristic ability regardless of the cause or means of such modification or mutation.

The HA-binding peptide of the present invention can be produced by the general technology for chemical synthesis based on its amino acid sequence. This technology includes the conventional liquid-phase and solid-phase methods. More particularly, the technology of peptide synthesis includes the stepwise elongation method in which the component individual amino acids are serially condensed one after another according to the amino acid sequence information provided by the present invention and the fragment condensation method which comprises synthesizing fragments each consisting of several amino acids in advance and coupling these fragments. The synthesis of peptides of the invention can be made by whichever of the above methods.

The condensation reactions for use in this peptide synthesis may also be carried out by various known methods. Specific processes include but are not limited to the azide process, mixed acid anhydride process, DCC process, active ester process, redox process, DPPA (diphenylphosphoryl azide) process, DCC + additive (1-hydroxybenzotriazole, N-hydroxysuccinimide, N-hydroxy-5-norbornene-2,3-dicarboximide, or the like) process, and Woodward process. The solvent for use in these processes can also be judiciously selected from among the common solvents which are well known to be of use for peptide condensation reactions of this kind. Specifically, there can be mentioned dimethylformamide (DMF), dimethyl sulfoxide (DMSO), hexaphosphoramide, dioxane, tetrahydrofuran (THF), ethyl acetate, and mixtures of such solvents, to mention but a few examples.

In conducting the above reactions for peptide synthesis, the carboxyl groups of amino acids or fragment peptides which are not to be involved in the reactions can be generally protected by esterification, for example in the form of a lower alkyl ester, e.g. methyl ester, ethyl ester, tert-butyl ester or the like, or an aralkyl ester such as benzyl ester, p-methoxybenzyl ester, p-nitrobenzyl ester or the like. In the case of an amino acid having a functional group as a side chain, the hydroxyl group of Tyr being an example, such functional group may be protected with a suitable protective group such as acetyl, benzyl, benzyloxycarbonyl, tert-butyl or the like but such protection is not always essential. To cite a further example, the guanidino group of Arg, for instance, can be protected with a suitable protective group such as nitro, tosyl, 2-methoxybenzenesulfonyl, methylene-2-sulfonyl, benzyloxycarbonyl, isobornyloxycarbonyl, adamantyloxycarbonyl or the like. The reactions for elimination of protective groups from such protected amino acids or peptides or from the end product peptide of the invention can also be carried out by the routine technology, for example by catalytic reduction or by a method using liquid ammonia/sodium, hydrogen fluoride, hydrogen bromide, hydrogen chloride, trifluoroacetic acid, acetic acid, formic acid or methanesulfonic acid, to mention but a few examples.

The HA-binding polypeptide of the invention, thus obtained, can be purified as needed in the routine manner, that is to say by using the methods in common use in the field of peptide chemistry, such as ion exchange resin partition chromatography, gel chromatography, affinity chromatography, high performance liquid chromatography (HPLC), counter-current distribution, and so forth.

Further, the above HA-binding peptide of the invention can be suitably modified. For example, by chemical modification such as alkylation or "lipidation" (phospholipidation), the cell affinity or tissue affinity of the HA-binding peptide can be enhanced and/or the blood half-life time of the peptide be increased to thereby potentiate its pharmacologic activity.

Alkylation of the HA-binding peptide can be carried out in the routine manner. For example, this can be easily done by an amide-forming reaction between a fatty acid and the N-terminal amino group of the HA-binding peptide (under the same conditions as already described for peptide synthesis).

The fatty acid can be liberally selected, without any particular restriction, from a broad range of compounds, regardless of whether it is a straight-chain acid or a branched-chain acid and whether it is saturated or unsaturated. Generally, fatty acids occurring in the living body can be chosen with advantage. Thus, fatty acids containing about 12∼20 carbon atoms, i.e. such saturated fatty acids as lauric acid, myristic acid, palmitic acid, stearic acid, arachic acid, etc. and such unsaturated fatty acids as oleic acid, eraidic acid, rinolic acid, linolenic acid, arachidonic acid, etc. can be mentioned by way of example.

Said alkylation may also be effected by the amide-forming reaction between an alkylamine and the C-terminal carboxyl group of the HA-binding peptide (under the same conditions as already described for peptide synthesis). As the alkylamine, various alkylamines can be used as in the case of physiological fatty acids mentioned above, and generally those having fatty acid chains (about 12∼20 carbon atoms ) which exist physiologically can be used with advantage.

The lipidation of the HA-binding peptide can also be carried out in the conventional manner (New Current, 11(3), 15-20 (2000); Biochemica et Biophysica Acta., 1128, 44-49 (1992); FEBS Letters, 413, 177-180 (1997); J. Biol. Chem., 257, 286-288 (1982), etc.). For example, utilizing the 2-hydroxyl or 3-phosphoric group of a phospholipid, a lipid-modified HA-binding peptide can be constructed through an arbitrary spacer. For this reaction, various condensation techniques can be employed and, where necessary, a cysteine-containing amino acid sequence of arbitrary length (usually several residues) can be added to the N- or C-terminus of the HA-binding peptide to introduce the reactive SH group useful for condensation.

The phospholipid mentioned above is not particularly restricted, either. Thus, for example, the compounds derived from said various fatty acids, such as phosphatidic acid, phosphatidylcholine (lecithin), phosphatidylethanolamine, phosphatidylserine, phosphatidylinositol, phosphatidylglycerol, etc. can be used with success.

The alkylated or lipidated HA-binding peptide (lipopeptide) according to the invention functions as a lipid component in the preparation of a liposome as well and, further, when the peptide is presented on the liposome, it can be used with great advantage as the liposome preparation to be described hereinafter.

The HA-binding peptide of the invention has an amino acid sequence which specifically recognizes and binds the influenza virus hemagglutinin involved in the first step of influenza infection and, by itself, does competitively antagonize the binding of the hemagglutinin-mediated binding of the influenza virus to the host cell receptor in vivo, thus being of use as a hemagglutinin binding inhibitor. This hemagglutinin binding inhibitor can be used as a tool for casting more light on the hemagglutinin-mediated influenza virus infection and the associated various cell functions and vital phenomena. Furthermore, it is expected to be of use as an antiinfluenza drug for preventing an influenza virus infection arising from the binding of the viral hemagglutinin to the host cell receptor or curing influenza, that is to say as the active ingredient of an antiinfective drug for influenza or a therapeutic drug for influenza.

The influenza virus to which the present invention is directed is not particularly restricted in type or origin as mentioned hereinbefore and may be any of type A, type B and type C viruses or a virus of the human or other animal origin, e.g. swine or equine origin, or even of the avian origin. The preferred are influenza A viruses. Also preferred are viruses of the human origin or human-infective viruses.

For use as an active ingredient in a prophylactic or therapeutic composition for influenza virus infection, the HA-binding peptide (inclusive of a mutated or modified peptide derived therefrom and having an HA-binding affinity; the same applies hereinafter) according to the invention is administered to a recipient individual, either as it is or in the form of a pharmaceutical composition containing a pharmaceutically acceptable carrier.

The pharmaceutically acceptable carrier mentioned above can be judiciously selected from among the carriers in routine use in the art according to the specific form of the pharmaceutical composition.

By way of illustration, when the pharmaceutical composition is to be prepared in the form of an aqueous solution, purified water (sterile water) or a physiological buffer solution can be utilized as said carrier. Moreover, when the composition is to be provided in the form of a suitable solution, a glycol, a glycerol or an injectable organic ester, such as olive oil, can also be used as said carrier. Moreover, the composition may be supplemented with a stabilizer, excipient and other additives which are in routine use in the field of peptide preparations or protein preparations.

The HA-binding peptide of the invention can also be provided in the form of a liposome preparation.

The liposome preparation can be produced by causing the HA-binding peptide of the invention to be supported on a liposome comprising an acid phospholipid as membrane constituent or a neutral phospholipid and an acid phospholipid as membrane constituents.

The acid phospholipid as a membrane constituent is defined here in a narrower sense than the ordinary definition of acid phospholipid. Specifically, there can be mentioned native or synthetic phosphatidylglycerols (PG) such as dilauroylphosphatidylglycerol (DLPG), dimyristoylphosphatidylglycerol (DMPG), dipalmitoylphosphatidylglycerol (DPPG), distearoylphosphatidylglycerol (DSPG), dioleoylphosphatidylglycerol (DOPG), egg yolk phosphatidylglycerol (egg yolk PG), hydrogenated egg yolk phosphatidylglycerol, etc. and native or synthetic phosphatidylinositols (PI) such as dilauroylphosphatidylinositol (DLPI), dimyristoylphosphatidylinositol (DMPI), dipalmitoylphosphatidylinositol (DPPI), distearoylphosphatidylinositol (DSPI), dioleoylphosphatidylinositol (DOPI), soybean phosphatidylinositol (Soybean PI), hydrogenated soybean phosphatidylinositol, and so forth. These may be used each independently or as a mixture of two or more species.

The neutral phospholipid includes native and synthetic phosphatidylcholines (PC) such as soybean phosphatidylcholine, egg yolk phosphatidylcholine, hydrogenated soybean phosphatidylcholine, hydrogenated egg yolk phosphatidylcholine, dimyristoylphosphatidylcholine (DMPC), dipalmitoylphosphatidylcholine (DPPC), dilauroylphosphatidylcholine (DLPC), distearoylphosphatidylcholine (DSPC), myristoylpalmitoylphosphatidylcholine (MPPC), palmitoylstearoylphosphatidylcholine (PSPC), dioleoylphosphatidylcholine (DOPC), etc, andnative and synthetic phosphatidylethanolamines (PE) such as soybean phosphatidylethanolamine, egg yolk phosphatidylethanolamine, hydrogenated soybean phosphatidylethanolamine, hydrogenated egg yolk phosphatidylethanolamine, dimyristoylphosphatidylethanolamine (DMPE), dipalmitoylphosphatidylethanolamine (DPPE), dilauroylphosphatidylethanolamine (DLPE), distearoylphosphatidylethanolamine (DSPE), myristoylpalmitoylphosphatidylethanolamine (MPPE), palmitoylstearoylphosphatidylethanolamine (PSPE), dioleoylphosphatidylethanolamine (DOPE) and so forth. These may be used each independently or as a mixture of two or more species.

The liposome membrane mentioned above can be formed in the conventional manner using said acid phospholipid as the sole constituent or said neutral phospholipid and acid phospholipid in combination. The proportion of the acid phospholipid relative to the whole composition of the liposome membrane is about 0.1∼100 mole %, preferably about 1∼90 mole %, more preferably about 10∼50 mole %.

In preparing said liposome, a further component material such as, for example, cholesterol can be added. By the addition of cholesterol, the fluidity of the phospholipid can be modulated to facilitate production of the liposome. The cholesterol is added at a level up to one equivalent, preferably at a level of 0.5∼1 equivalent, relative to the phospholipid.

The formulating ratio of the acid phospholipid to the active ingredient in a liposome dispersion is about 0.5∼100 equivalents, preferably about 1∼60 equivalents, more preferably about 1.5∼20 equivalents, relative to the active ingredient.

The amount of the HA-binding peptide to be contained in the liposome may be a few mole % to tens of mole %, preferably about 5∼10 mole %, based on the total lipid, although it may generally be about 5 mole %.

For the production of a liposome preparation containing the HA-binding peptide of the invention as the drug, various known technologies can be employed. Moreover, the peptide of the invention, when it is a lipopeptide, can be processed into the desired liposome by utilizing it as the lipid component in such technologies.

For example, multi-lamellar liposomes (MLV) can be produced as follows. First, a lipid is dissolved in an organic solvent (chloroform, ether or the like) and placed in a round-bottom flask and the organic solvent is removed under nitrogen or reduced pressure to leave a thin lipid membrane in the bottom of the flask. In this step, the membrane may be further left standing under reduced pressure in a desiccator so as to completely remove the organic solvent. Then, a drug solution is added onto the thin lipid membrane and the lipid is hydrated to give an opal white liposome suspension.

Large unilamellar liposomes (LUV) can be produced by the procedure which comprises adding Ca²⁺ to small unilamellar liposomes of phosphatidylserine to prepare a cylindrical sheet by fusion and adding the chelating agent EDTA so as to remove Ca²⁺ (Biochim. Biophys. Acta 394, 483-491, 1975) or the method which comprises pouring an ether solution of a lipid into an aqueous medium at about 60° C and evaporating off the ether (Biochim. Biophys. Acta 443, 629-634, 1976).

The method of preparing liposomes by reverse-phase technique devised by Szoka et al. (Proc. Natl. Acad. Sci. U.S.A. 75, 4194-4198, 1978) can also be employed. In this method, a drug solution is added to an ether solution of a phospholipid and the mixture is sonicated to give a W/O emulsion. This W/O emulsion is subjected to an evaporator treatment under reduced pressure to remove the ether and a buffer solution is then added. The resulting mixture is stirred with a vortex mixer, whereupon the W/O emulsion is reversed into an O/W emulsion, and the residual organic solvent is removed to give liposomes.

Aside from the above production technologies, liposomes of small vesicle size can be prepared by the French press technique (FEBS Lett. 99, 210-214, 1979). It is also possible to use freeze-drying (Chem. Pharm. Bull. 32, 2442-2445, 1984) and freeze-thaw (Chem. Pharm. Bull. 33, 2916-2923, 1985) techniques which feature high liposome-entrapping efficiencies as reported by Ohsawa et al.

The liposomes thus prepared can be size-selected by dialysis (J. Pharm. Sci. 71, 806-812, 1982) or a filtration technique using a polycarbonate membrane (Biochim. Biophys. Acta 557, 9-23, 1979; Biochim. Biophys. Acta 601, 559-571, 1980). Moreover, a dialytic technique, a gel filtration technique or a centrifugal technique can be used for removing the drug not entrapped by the liposomes from the liposome solution (Liposome: "Chemistry of Lipids [Society of Japan(ed.), Seikagaku Jikken Koza (Biochemical Experiment Series) 3], Tokyo Kagaku Dojin, 1974). Furthermore, the liposomes can be concentrated by means of a dialysis membrane.

The liposome dispersion thus produced may be supplemented with various known additives as needed for drug design, such as the antiseptic, isotonizing agent, buffer, stabilizer, solubilizer, adsorption promoter, etc. in suitable amounts, and where necessary, can be diluted with a liquid containing such additives or water. The additives mentioned above include the following specific substances, among others. The antiseptic includes preservatives which are active against fungi and bacteria, such as benzalkonium chloride, benzethonium chloride, chlorhexidine, parabens (methyl paraben, ethyl paraben, etc.), thimerosal, etc.; the isotonizing agent includes polyhydric alcohols such as D-mannitol, D-sorbitol, D-xylitol, glycerol, glucose, maltose, sucrose, propylene glycol, etc. and electrolytes such as sodium chloride; the stabilizer includes tocopherol, butylhydroxyanisole, butylhydroxytoluene, ethylenediaminetetraacetic acid (EDTA), cysteine, and so forth.

Further, internally of said liposome comprising the HA-binding peptide of the invention may be further incorporated one or more other drugs such as an antiviral agent to provide a liposome preparation in the like manner.

Liposome preparations can be manufactured by the procedures described in Woodle et al. (Long Circulating Liposomes: old drugs, New therapeutics., M. C. Woodle, G. Storm, Eds: Springer-Verlag Berlin (1998)) and Namba et al. (Liposomal applications to cancer therapy, Y. Namba, N. Oku, J. Bioact. Compat. Polymers, 8, 158-177(1993)), among other techniques. A typical liposome preparation relevant to the present invention is disclosed hereinafter in Example 9.

The amount of the HA-binding peptide in the pharmaceutical composition inclusive of said liposome preparation according to the invention is not particularly restricted but can be liberally selected from a broad range. Usually, the amount of the peptide may be about 0.0002∼0.2% (w/v %), preferably about 0.001∼0.1 (w/v %), based on the whole composition.

The influenza virus hemagglutinin binding inhibitor and pharmaceutical composition of the present invention can each be administered in the form of a pharmaceutical preparation containing the HA-binding peptide as an active ingredient in a pharmaceutically acceptable carrier to individuals either after or before influenza virus infection.

The method of administering said inhibitor or pharmaceutical composition is not particularly restricted but can be judiciously selected according to the dosage form, patient factors such as age, sex, etc., and severity of illness, among other variables. The preferred dosage form includes non-oral dosage forms such as injections, drip injections, nasal drops, and inhalants. Particularly when the dosage form is an injection or a drip injection, it is administered intravenously, optionally in admixture with a standard infusion such as a glucose infusion or an amino acid infusion or administered intramuscularly, intradermally, subcutaneously or intraperitoneally.

The daily dosage of the influenza virus hemagglutinin antagonist (binding inhibitor) or pharmaceutical composition of the present invention cannot be stated in general terms, for it depends on the recipient's condition, body weight, age, sex and other factors but, in terms of the amount of the HA-binding peptide, the daily dosage can be chosen from the range of about 0.001∼100 mg per adult human. The preparation of the invention can be administered not only in a single daily dose but also in several divided doses a day.

### BEST MODE FOR CARRYING OUT THE INVENTION

The following examples are intended to illustrate the present invention and should by no means be construed as defining the technical scope of the invention. The present invention may be easily modified and altered by those skilled in the art on the basis of the disclosure in this specification without departing from the technical purview of the invention.

### Reference Example 1

### Construction of a phage display library

A phage display library (2.5×10⁸ clones) was constructed by reference to the report of Nishi, Saya, et al. (Nishi T., Saya H., et al., FEBS Lett, 399, 237-240 (1996)). This phage display library is a filamentous phage fd in which a DNA containing a sequence of 15 repeats of NNK (N represents the nucleotide adenine (A), cytosine (C), guanine (G) or thymine (T) and K represents guanine (G) or thymine (T)) has been inserted by a genetic engineering technique and has been so constructed that a DNA coding for a random 15-residue amino acid sequence is inserted in the N-terminal region of the capsid protein pIII gene so that the peptide having a random 15-residue amino acid sequence may be expressed on the phage capsid surface.

The above phage display library has the features reported by Scott et al. (Scott. J. K. and Smith, G. P., Science, 249, 386-390 (1990)).

### Reference Example 2

### Immobilization of an influenza virus hemagglutinin

For use as the target of a biopanning, the influenza virus hemagglutinin was immobilized as follows.

As the hemagglutinin (HA) to be immobilized, an ether extract of the type A subtype H1 A/PR/8/34 (H1N1) (hereinafter referred to sometimes briefly as H1 or H1N1) and an ether extract of the type A subtype H3 (A/Wuhan/359/95 (H3N2) (hereinafter referred to sometimes briefly as H3 or H3N2) were used.

In the first place, 60 µl of a 1:1 mixture of EDC (1-athyl-3-(3-dimethylaminopropyl)carbodiimide) and N-hydroxysuccinimide was added to each well of a 96-well carboplate (product of Sumilon) to activate the carboxyl group bound to the plate. After 10 minutes, the wells of the plate were washed with 50 mM TBS buffer (pH 7.6) 6 times and 100 µl of a solution of H1N1 (70 µl/ml) or a solution of H3N2 (70 µl/ml) was added. The plate was allowed to sit for 2 hours, after which time the wells were washed with 50 mM TBS buffer (pH 7.6) 6 times, and 100 µl of 1 mM ethanolamine/H₂O was then added. The plate was allowed to sit for 10 minutes, after which the wells were washed again with 6 portions of 50 mM TBS buffer (pH 7.6) to complete immobilization of the influenza virus HA on the plate (Fig. 1).

### Example 1

### Selection and identification of an influenza HA (H1)-binding peptide

### (1) Selection of HA-binding peptide display phages

### (i) Biopanning -first cycle-

Using the HA (H1N1) plate prepared in Reference Example 2 as the HA-supporting plate, 100 µl of the phage display library solution prepared in Reference Example 1 (6.2×10¹⁰ TU/TBS buffer) was injected and the plate was allowed to sit at room temperature for 4 hours. The plate was then washed with good pipetting 5 times using 200 µl each of an elution buffer (50 mM Tris-HCl, 150 mM NaCl) to remove the unbound non-specific phages. Then, 100 µl of 10 mM sialic acid derivative 7-F-Neu5Ac2en solution or 10 mM ganglioside GM3 solution was added to each well and the plate was allowed to sit at room temperature for 1 hour.

It has been confirmed by the present inventors that the sialic acid derivative 7-F-Neu5Ac2en is an HA inhibitor having the ability to inhibit binding of hemagglutinin, particularly binding of the sialic acid-containing sugar chain to H1N1, while the ganglioside GM3 is a specific HA receptor substance having a specific binding affinity for the receptor-binding pocket of hemagglutinin [T. Saito, et al., Chem. Lett., 145 (1999)]. Therefore, by adding said HA inhibitor or HA receptor substance to the wells of a phage-supporting plate, the phage specifically bound to the hemagglutinin receptor binding pocket of the influenza virus can be eluted.

The HA-binding phage was eluted in the above manner and a solution containing the phage was recovered.

To the phage solution obtained as above was added 100 µl of a prepared solution of E. coli K91-Kan for 15-minute infection at room temperature. After 15 minutes, this infected solution was added to 20 ml of NZY medium containing 0.2 µg/ml of tetracycline (product of Sigma) (hereinafter referred to as TC) which had been warmed to 37°C in advance and shake-cultured at 37°C for 40 minutes.

After 40 minutes, 20 µl of a TC stock solution (20 mg/ml) was added (final concentration 20 µg/ml) and the shake culture was continued at 37° C overnight. The resulting culture was centrifuged at 3,000 rpm for 10 minutes to remove E. coli. The supernatant was further centrifuged at 12,000 rpm for 10 minutes to thoroughly remove E. coli. To the supernatant thus obtained was added 3 ml (0.15 v/v) of PEG/NaCl solution, and after 100 times of gentle stirring, the mixture was allowed to stand at 4°C for not less than 4 hours. The mixture was centrifuged at 12,000 rpm for 10 minutes to remove the supernatant and the phage pellet obtained was dissolved thoroughly by adding 1 ml of TBS. This phage solution was transferred to a 1.5 ml Eppendorf tube (Ep tube) and centrifuged at 15,000 rpm for 10 minutes to remove the insolubles. To this solution was added 150 µl of PEG/NaCl solution again, and after several cycles of gentle stirring, the mixture was allowed to stand at 4°C for not less than 1 hour. The mixture was then centrifuged at 15,000 rpm for 10 minutes to recover a pellet, which was then dissolved in 0.02% NaN₃/TBS (200 µl). This solution was centrifuged at 15,000 rpm for 10 minutes to remove insolubles and the supernatant phage solution (200 µl) was transferred to a 0.6 ml Ep tube. One-half of the phage solution thus obtained was submitted to the second and subsequent panning cycles, a 2 µl portion was used for titering, and the remainder was stored.

### (ii) Biopanning -second cycle-

The HA (H1N1)-supporting plate prepared in Reference Example 2 was injected with 100 µl of an amplified phage display library solution and the plate was allowed to sit at room temperature for 4 hours. Then, using 200 µl of an elution buffer (50 mM Tris-HCl, 150 mM NaCl), the wells were thoroughly washed 10 times with good pipetting to remove the unbound non-specific phages. Thereafter, 100 µl of 10 mM sialic acid derivative 7-F-Neu5Ac2en solution or 10 mM GM3 solution was added to each well and the plate was allowed to sit at room temperature for 1 hour. The phage specifically bound to the influenza HA binding pocket was thus eluted and a solution containing the phage was recovered.

### (iii) Biopanning -third cycle-

The HA (H1N1)-supporting plate was injected with 100 µl of an amplified phage display library solution and the plate was allowed to sit at room temperature for 1 hour. Then, the wells were thoroughly washed with 200 µl of an elution buffer (50 mM Tris-HCl, 300 mM NaCl) 10 times with good pipetting to remove the unbound non-specific phages . Then, 100 µl of 10 mM sialic acid derivative 7-F-Neu5Ac2en solution or 10 mM GM3 solution was added to each well and the plate was allowed to sit at room temperature for 1 hour. The phage specifically bound to the receptor-binding pocket of the influenza HA was thus eluted and a solution containing the phage was recovered.

### (iv) Biopanning -fourth cycle-

The HA (H1N1)-supporting plate was injected with 100 µl of an amplified phage display library solution and the plate was allowed to sit at room temperature for 1 hour. Then, the wells were thoroughly washed with 200 µl of an elution buffer (50 mM Tris-HCl, 300 mM NaCl) 10 times with good pipetting to remove the unbound non-specific phages . Then, 100 µl of 10 mM sialic acid derivative 7-F-Neu5Ac2en solution or 10 mM GM3 solution was added to each well and the plate was allowed to sit at room temperature for 30 minutes. The phage weakly bound to the receptor-binding pocket of the influenza HA was thus eluted. Then, 100 µl of 10 mM sialic acid derivative 7-F-Neu5Ac2en solution or 10 mM GM3 solution was added to each well and the plate was allowed to sit at room temperature for 1 hour. The phage specifically bound to the binding pocket of HA was thus eluted and a solution thereof was recovered.

### (v) Biopanning -fifth ∼ seventh cycles-

The procedure of (iv) was repeated for the 5th, 6th and 7th cycles of biopanning.

The results (phage recovery rates) obtained by the above cycles of biopanning (first ∼ seventh cycles) are shown, by kind of eluent (7-F-Neu5Ac2en or GM3), in Tables 1 and 2.

**Table 1**

| <H1-7F> Immobilized virus: A/PR/8/34 (H1N1) Eluent: sialic acid derivative 7-F-Neu5Ac2en | | | |
|---|---|---|---|
| Panning cycle | Amount of phage used | Amount of phage recovered | Phage recovery rate |
| | (TU) | (TU) | (%) |
| 1st | 6.2×10¹⁰ | 7.7×10⁷ | 0.12 |
| 2nd | 2.6×10¹² | 1.1×10⁷ | 0.0044 |
| 3rd | 2.6×10¹³ | 2.9×10⁸ | 0.0011 |
| 4th | 8.7×10¹³ | 8.7×10⁸ | 0.0010 |
| 5th | 1.7×10¹³ | 2.5×10⁹ | 0.015 |
| 6th | 5.1×10¹¹ | 2.8×10⁹ | 0.55 |
| 7th | 1.5×10¹² | 8.2×10⁸ | 0.055 |

**Table 2**

| <H1-GM3> Immobilized virus: A/PR/8/34 (H1N1) Eluent: ganglioside GM3 | | | |
|---|---|---|---|
| Panning cycle | Amount of phage used | Amount of phage recovered | Phage recovery rate |
| | (TU) | (TU) | (%) |
| 1st | 6.2×10¹⁰ | 8.8×10⁷ | 0.14 |
| 2nd | 1.8×10¹² | 1.8×10⁷ | 0.0010 |
| 3rd | 3.6×10¹² | 1.1×10⁸ | 0.0030 |
| 4th | 5.7×10¹³ | 2.1×10⁹ | 0.0037 |
| 5th | 2.9×10¹³ | 2.1×10¹⁰ | 0.072 |
| 6th | 5.0×10¹¹ | 7.1×10⁹ | 0.14 |
| 7th | 1.6×10¹² | 7.4×10⁸ | 0.046 |

In either case, whereas no remarkable gains were obtained in the recovery rate from the first to the fourth cycle because of the stepwise-increasing severity of panning conditions, the phage recovery rate increased from the fourth to the six cycle where the panning was repeated under uniform conditions. It could, thus, be confirmed that phages expressing the peptide specifically binding to the influenza virus hemagglutinin (H1N1) were recovered.

### (2) Determination of the amino acid sequence of an HA-binding peptide

In the above panning procedure, the phage obtained by the sixth cycle of panning was used to determine the amino acid sequence of the expressed peptide, specifically, two kinds of phages, namely the phage (hereinafter referred to as H1/7 phage: 19 clones) bound to the immobilized hemagglutinin H1N1 and eluted with 7-F-Neu5Ac2en and the phage (hereinafter referred to as H1/3 phage: 20 clones) bound to the immobilized hemagglutinin H1N1 and eluted with ganglioside GM3, were used for the sequencing of the expressed peptides.

First, from among the colonies on the plates obtained in the titering after the 6th panning cycle, 50 colonies each were picked up at random, reinoculated on a fresh NZY plate and cultured at 37°C overnight. This was stored as a master plate at 4°C. The colonies on each master plate were suspended in a 50 ml centrifuge tube containing 20 ml of NZY medium (supplemented with 20 µg/ml of TC) and shake-cultured at 37°C and 200 rpm overnight. The resulting culture was centrifuged at 3,000 rpm for 10 minutes and the supernatant was transferred to an Oak-Ridge centrifuge tube in which it was centrifuged at 12,000 rpm for 10 minutes to remove E. coli. The supernatant was further transferred to an Oak-Ridge centrifuge tube, in which 3 ml of polyethylene glycol (PEG 6000; product of Nacalai Tesque)/NaCl was added and the mixture was stirred well and then allowed to stand at 4°C for 4 hours. The mixture was centrifuged at 12,000 rpm for 10 minutes to precipitate the phages. The supernatant was discarded and the phage pellet was suspended in 1 ml of TBS (Tris-buffer solution). The suspension was transferred to a 1.5 ml Ep tube and centrifuged at 15,000 rpm for 10 minutes to remove insolubles. The supernatant was transferred to another Ep tube, in which 150 µl of polyethylene glycol/NaCl was added. The mixture was stirred well and, then, allowed to stand at 4°C for 1 hour. It was then centrifuged at 15,000 rpm for 10 minutes to reprecipitate the phages. The supernatant was discarded and the phage pellet was resuspended in 200 µl of TBS. The suspension was centrifuged at 15,000 rpm for 10 minutes to precipitate insolubles and the precipitate was transferred to a 0.5 ml Ep tube. The phage clone was stored at 4°C.

To extract DNA from the phage clone obtained as above, 100 µl of TBS and 200 µl of TE-saturated phenol (product of Nippo Gene) per 100 µl of the phage clone were added to the 1.5 ml Ep tube and the mixture was vigorously stirred for 10 minutes and, then, centrifuged at 15,000 rpm for 10 minutes. To 200 µl of the resulting supernatant (aqueous phase) were added 200 µl TE-saturated phenol and 200 µl of chloroform, and the mixture was stirred vigorously for 10 minutes in the same manner as above and centrifuged at 15,000 rpm for 10 minutes. Then, to 150 µl of the supernatant (aqueous phase) were added 250 µl of TE, 40 µl of 3 M sodium acetate, 1 µl of 20 mg/ml glycogen (Boehringer-Mannheim) and 1 ml of ethanol. The mixture was allowed to stand in a 1.5 ml Ep tube at -20° C for 1 hour and, then, centrifuged at 15,000 rpm for 10 minutes. The supernatant was discarded, 1 ml of 80% ethanol (-20°C) was added slowly, and the mixture was centrifuged at 15,000 rpm for 10 minutes to remove the residual salt. The supernatant was discarded and the water in the tube was evaporated off. The DNA pellet was dissolved in 10 µl of sterile distilled water and stored at 4°C. The phage DNAs obtained in the above manner were used for peptide sequencing.

The sequencing of the peptide encoded by phage DNA was determined by the dideoxy method (Proc. Natl. Acad. Sci., USA., 74, 5463-5467 (1977)) using Amersham's THERMO Sequencing Kit (Amersham life Science, Code; US79765, Lot: 201503) according to the accompanying manual. The PCR reaction of the DNA was carried out in 30 cycles of 96°C, 30 sec., 45°C, 15 sec., 60°C, 4 min. and the DNA sequencing was made using ABI's DNA sequencer (ABI PRISMTM377).

The amino acid sequences of the peptides having a binding affinity for the receptor-binding pocket of the hemagglutinin as determined from clones of the two kinds of phages (H1/7 phage and H1/3 phage) are shown in one-letter expression in Table 3. The amino acid sequences indicated in the following table are restated hereinafter under SEQUENCE LISTING as SEQ ID NO:1 (NOs: 1 and 5), 2 (NO:2), 3 (NO:3), 4 (NO:4), 5 (NO:6) and 6 (NO:7), respectively.

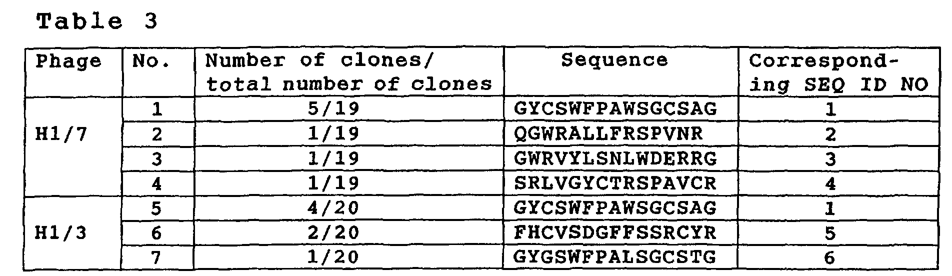

Referring to each phage, the remaining clones not given in the Table either showed random defects in sequences or could not be sequenced.

It can be seen from the Table that both phage H1/7 and phage H1/3 had an amino acid sequence in common between a plurality of clones. Furthermore, comparison of the two phages revealed a common sequence between H1/7 and H1/3 (No. 1 and No. 5).

The HA receptor substance ganglioside GM3 is known to have a high affinity for hemagglutinin H1 and the HA inhibitor 7F-Neu5Ac2en is known to have a high antagonist activity against hemagglutinin H1.

### Example 2

### Binding affinity of the HA-binding peptide for influenza hemagglutinin

The phage obtained in Example 1 was cloned and the binding affinity of the expressed peptide for hemagglutinin was evaluated by ELISA.

First, the hemagglutinin (H1N1) was immobilized on a 96-well carboplate (product of Sumilon) in accordance with the procedure described in Reference Example 2. After immobilization, 100 µl/well of TBS containing 1% BSA (Albumin Bovine, Fatty Acid Free; Sigma) was added to the plate and the plate was allowed to sit at room temperature for 1 hour. To each well was added 50 µl of an amplified phage solution (H1/H7 phage: Clone Nos. 1∼4; H1/3 phage: Clone Nos. 5∼7), and the plate was allowed to sit at room temperature for 1 hour. After each well was washed 5 times with TBST (TBS/5% Tween 20), 150 µl of TBS/5% BSA was added, and the plate was allowed to sit at room temperature overnight.

Then, 150 µl/well of anti-fd bacteriophage antibody/TBS solution (1/1000 dilution) was added and the plate was allowed to sit at room temperature overnight. The wells were washed with 5 portions of TBST. Then, 100 µl/well of anti-rabbit IgG peroxidase conjugate antibody/TBS solution (1/1000 dilution) was added and the plate was allowed to sit at room temperature overnight and, then, washed with 5 portions of TBST.

The substrate solution (0.4 mg/ml o-phenylendiamine substrate in citrate-phosphate buffer (pH 5.0)) was added, 100 µl/well, and the reaction was carried out for 10 minutes. The reaction was stopped by adding 100 µl/well of 3 N-H₂SO₄ and the absorbance at 492 nm was measured with a microplate reader.

Using the H1 phages (H1/7 phage and H1/3 phage) subjected to panning with hemagglutinin (H1N1) as the target, the binding affinity for hemagglutinin H1 was investigated. The results are presented in Fig. 2.

It will be apparent from Fig. 2 that Clone No. 1 of H1/7 phage (indicated by ○ in Fig. 2; hereinafter referred to as H1-1) and Clone No. 6 of H1/3 phage (indicated by △ in Fig. 2 ; hereinafter referred to as H1-2) showed high binding affinities for hemagglutinin H1.

This result indicates that phages expressing peptides binding specifically to the recognition site (receptor-binding pocket) of hemagglutinin could be selected by the invention.

### Example 3

### Selection of an HA-binding peptide recognizing two subtypes

Biopanning was carried out as in Example 1 using two kinds of influenza virus hemagglutinins H1 (H1N1, A/PR/8/34) and H3 (H3N2, A/Wuhan/359/95) as the target. Specifically, using the H1-supporting plate and H3-supporting plate prepared in accordance with Reference Example 2 as the HA-supporting plate and the HA receptor Sialyl-LewisX and α (2→6) GM3 as the eluent, biopanning was performed in the same manner as in Example 1 to select phages expressing HA-binding peptides. For representative phage clones obtained by the biopanning, the amino acid sequences of the expressed peptides were determined. The results are shown in Table 4.

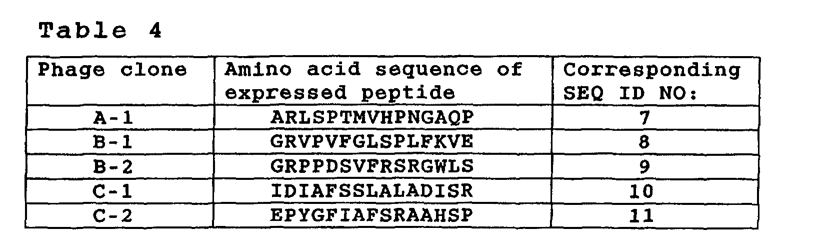

Clone "A-1" was found among the phage clones selected by all the panning systems shown below in Table 5.

**Table 5**

| Panning system | Target HA | Eluent | Number of A-1 clones/ Total number of clones |
|---|---|---|---|
| H1/X | H1N1 | Sialyl-LewisX | 2/10 |
| H1/GM | H1N1 | α(2→6)GM3 | 2/9 |
| H3/X | H3N2 | Sialyl-LewisX | 2/10 |
| H3/GM | H3N2 | α(2→6)GM3 | 9/10 |

Clone "B-1" was found in two of 9 clones selected by the panning system "H1/GM".

Clone "B-2" was found in one of 10 clones selected by the panning system "H3/X".

Clone "C-1" was found in one of 9 clones selected by the panning system "H1/GM" and in one of 10 clones selected by the panning system "H3/GM".

Clone "C-2" was found in one of 9 clones selected by the panning system "H1/GM" and in 4 of 10 clones selected by the panning system "H3/X".

It is clear from the above results that Clone "A-1" was selected from all the panning systems varying in target and eluent. The finding that clones expressing peptides of the same sequence could be obtained by pannings with different eluents suggests that the peptide expressed by this Clone "A-1" has the same hemagglutinin binding sites (NeuAc-associated sites) as the binding sites possessed by the HA receptor substances Sialyl-LewisX and a (2→6) GM3 and that it is a peptide having the same binding mode as the above receptor substances.

The peptides expressed by Clones "B-1" and "B-2" were found to have GRxP (x represents V or P) as a common motif and that the peptides expressed by Clones "C-1" and "C-2" were found to have IAFSxyA (x represents S or R; y represents L or A) as a common motif.

### Example 4

### The binding affinity of HA-binding peptides for influenza hemagglutinin

For the phage clones "H1-1", "H1-2", "A-1", "B-1", "B-2", "C-1" and "C-2" obtained in Examples 1 and 3, the binding affinity for hemagglutinin (H1, H3) was evaluated by ELISA as in Example 2.

Specifically, hemagglutinins H1 (H1N1) and H3 (H3N2) and wheat germ agglutinin (WGA), which is a kind of lectin, were respectively immobilized on a 96-well carboplate (product of Sumilon) and ELISA was carried out by adding 5×10¹⁰ virions/ml of each phage clone solution.

The results are presented in Fig. 3. It will be apparent from Fig. 3 that whereas the Clones "H1-1" and "H1-2" obtained in Example 1 were scarsely bound to hemagglutinin H3 but showed specific binding affinities for hemagglutinin H1, the Clones "A-1", "B-1", "B-2", "C-1" and "C-2" obtained in Example 3 were invariably bound to both hemagglutinins H1 and H3 in common.

It is known that hemagglutinins H1 and H3 are different from each other by 75% in amino acid sequence. Therefore, the peptides expressed by clones "A-1", "B-1", "B-2", "C-1" and "C-2" are considered to recognize a homologous sequence of the two hemagglutinins, i.e. H1 and H3, and be bound specifically to that site.

### Example 5

### The binding affinity of HA-binding peptides for anti-GM3 antibody

For the phage clones "H1-1", "H1-2", "A-1", "B-1", "B-2", "C-1" and "C-2" obtained in Examples 1 and 3, the binding affinity of each clone for anti-GM3 antibody was evaluated by ELISA as in Example 4. Specifically, for ELISA, the anti-GM3 antibody (M2590, Nippon Biotest Kenkyusho) was immobilized on a 96-well carboplate (product of Sumilon) and 5×10¹⁰ virions/ml of each phage clone solution was added.

The results are presented in Fig. 4. It can be seen from Fig. 4 (a) and (b) that whereas the clones "H1-1" and "H1-2" obtained in Example 1 were little bound to anti-GM3 antibody, the clones "A-1", "B-1" , "B-2", "C-1" and "C-2" obtained in Example 3 were invariably bound specifically to anti-GM3 antibody.

The above results suggested that the peptide expressed by each of the clones "A-1", "B-1", "B-2", "C-1" and "C-2" has a sequence mimicking the sialic acid-containing structure of GM3.

### Example 6

### The inhibition of binding of hemagglutinin to ganglioside GM3 by phage clones

An experimental study was conducted in the routine manner to see whether the binding of a hemagglutinin to ganglioside GM3 would be inhibited by the phage clones ("H1-1", "H1-2" , "A-1", "B-1", "B-2", "C-1" and "C-2") obtained in Examples 1 and 3. ["Methods for Ganglioside Study I, edited and authored by Suzuki Yasuo and Ando Susumu, Gakkai Publishing Center, 1995]. As the hemagglutinin, H1 (H1N1) and H3 (H3N2) were used.

Specifically, 10 µl of GM3 solution (8.5 µg/ml methanol) was added to a 96-well microtiter plate, followed by addition of 50 µl of 0.08% polyisobutyl methacrylate solution. After the solvent was evaporated off with a dryer, 50 µl of 1% BSA/TBS was added and the plate was allowed to sit at 4° C overnight. After the plate was washed with 3 portions of TBS, 50 µl of a solution containing a phage clone of predetermined concentration (Table 6) and hemagglutinin (in a concentration such that the system hemagglutinin concentration would be 30 nM) was added and the plate was allowed to sit at 20°C for 2 hours. After the plate was washed with 5 portions of TBS, the amount of hemagglutinin bound to the plate was detected with the antibody and the inhibitory activity was determined from the amount of decrease. The results (IC₅₀ values) are shown in Table 6. In the table, "nd" means "not detected".

**Table 6**

| Phage clone | IC₅₀/nM (virions/ml) | |
|---|---|---|
| | H1N1 | H3N2 |
| A-1 | 5.0 (3.0×10¹²) | 17 (1.0×10¹³) |
| B-1 | nd | nd |
| B-2 | 9.9 (6.0×10¹²) | 11 (6.8×10¹²) |
| C-1 | 77.0 (4.7×10¹³) | 73 (4.4×10¹³) |
| C-2 | 270.0 (1.6×10¹⁴) | nd |
| H1-1 | 12.0 (7.5×10¹²) | nd |
| H1-2 | 24.0 (1.4×10¹³) | nd |

It will be apparent from Table 6 that for phage clones "A-1", "C-1" and "B-2", the expressed peptides inhibited binding of both hemagglutinins H1 and H3. These results were correlated with the binding specificity of these phage clones for hemagglutinins.

### Example 7

### Inhibition of binding of phages to hemagglutinins by a lipid

To confirm that the phage clones ("A-1", "B-1", "B-2", "C-1", "C-2") obtained in Example 3 actually recognize the host receptor recognition sites of hemagglutinins (HA), it was investigated by ELISA as in Example 6 whether the binding of said phage clones to immobilized HA is inhibited by GM3 which is an HA receptor substance. As the hemagglutinin, H1N1 and H3N2 were respectively used. As control, similar experiments were performed using GM1 and GlcCer in lieu of GM3.

The results of an experiment on the binding inhibition by GM3 are presented in Fig. 5 and the results of an experiment on the inhibition by GM1 are presented in Fig. 6. On both diagrams, a) represents the results with H1N1 as the hemagglutinin and b) represents the results obtained with H3N2.

It is apparent from Fig. 5 that, with all the phage clones tested, their binding was inhibited when the hemagglutinin-specific ligand (HA-receptor substance) GM3 was added. In contrast, the binding of these phage clones to the hemagglutinin was not inhibited in the presence of GM1 (Fig. 6) or GlcCer (not shown), which has no specific hemagglutinin-binding activity.

The above findings suggested that the peptides expressed by these phage clones undergo specific interactions with the receptor recognition site of influenza virus hemagglutinin to thereby specifically inhibit the binding of the influenza virus to the host receptor (infection).

### Example 8

### Synthesis of the peptide expressed on the phage

The peptides expressed on the phage clones obtained in Examples 1 and 3 were synthesized in the following manner.

Each peptide was synthesized by the solid-phase method (Fmoc/NMP, HOBt method) by using a full-automatic peptide synthesizer (ACT357, manufactured by Advanced Chemtech) in accordance with the manufacturer's program [Fmoc: 9-fluorenylmethoxycarbonyl; NMP: N-methylpyrrolidone; HOBt: 1-hydroxybenzotriazole].

To 0.25 mmol of Fmoc-amino acid-Alko resin corresponding to the C-terminal amino acid, the Fmoc-amino acids corresponding to the second and subsequent amino acids from the C-terminus were sequentially reacted for chain extension according to a synthesizing program based on the amino acid sequence to be synthesized. For the production of a peptide in the form of a C-amide, an Fmoc-amino acid corresponding to the C-terminal amino acid was condensed to 0.25 mmol of Fmoc-NH-FAL resin according to a synthesizing program and, thereafter, Fmoc-amino acids corresponding to the second and subsequent amino acids from the C terminus were serially condensed for chain extension.

After completion of each reaction, the N-terminal Fmoc group was subjected to deprotection reaction according to the program. The peptide resin thus obtained was recovered in a polypropylene mini-column (product of Assist Co.), washed with methanol, and dried in vacuo. Thereafter, the peptide was excised off the resin by the following procedure and the side chains were deprotected. Thus, to each resin was added 2 ml of Reagent K (82.5% TFA, 5% phenol, 5% H₂O, 5% thioanisole, 2.5% ethanedithiol), and the reaction was carried out in a mini-column for 60 minutes. Then, the reaction mixture was dripped into 8 ml of cold diethyl ether to stop the reaction and, at the same time, precipitate the peptide. Furthermore, after the mini-column was washed with 2 ml of TFA, 5 ml of cold diethyl ether was further added and the mixture was centrifuged. The pellet was washed 4 times with 10 ml portions of diethyl ether and the peptide was solubilized with about 5 ml of 50% acetonitrile and lyophilized. The solubilization and lyophilization procedure was repeated twice to give the objective crude lyophilizate.

This was fractionated by reversed-phase high performance liquid chromatography (HPLC) using an octadecylated column (20 dia. × 250 mm, product of WC) to isolate the desired peptide. The resins and amino acid derivatives used in the above procedure were invariably the products of Watanabe Chemical Industries, Ltd.

Identification of each peptide isolated in the above manner was carried out by amino acid analysis and molecular weight determination by mass spectrometry.

The synthetic peptides having cysteine residues can be optionally cyclized. Taking the peptide expressed by clone "H1-1" as an example, this peptide was cyclized in the following manner.

First, 5.0 mg (3.2 µl) of the peptide was dissolved in 5 ml of 200 mM Tris-HCl buffer (pH 8.5)/TFE (4:1) (0.64 mM). After the reaction system was adjusted to pH 8.5 with aqueous sodium hydroxide solution, it was stirred vigorously in an oxygen atmosphere at room temperature so that the SH group was oxidized to form an intramolecular disulfide linkage. This reaction was monitored by HPLC. After about 30 hours, the reaction mixture was adjusted to pH not below 7 with 10% sodium hydroxide/H₂O to thereby stop the reaction. The product was detected as a single peak. After the reaction mixture was subjected to desalting with an electrodialyzer (Microacylizer G1, manufactured by Asahi Kasei Kogyo), it was lyophilized to give the cyclized peptide as a white powder [yield 401 mg (2.6 µmol), 82%].

### Example 9

### Lipopeptides and peptide-modified liposomes

By coupling stearic acid to the N-terminus of the peptide, the HA-binding peptide of the invention can be introduced onto liposomes. The peptide expressed by the Clone "A-1" obtained in Example 3 is taken as an example in the following explanation.

First, the peptide expressed by clone "A-1" (SEQ ID NO:7) was synthesized by the cycling in accordance with the procedure of Example 8. Then, according to said synthesizing program, stearic acid was coupled to the N-terminus of the above peptide. The peptide was excised from the resin and deprotected. Thereafter, the procedure described in Example 8 was followed to give the desired stearic acid-coupled peptide ("C₁₈A-1 lipopeptide").

In a 30 ml egg plant-shaped flask, the above lipopeptide (Mw: 1840.73), egg yolk lecithin (Mw: 773, Nippon Oil and Fats, NC-10S) and cholesterol (Mw: 386.66; Nakarai Chemicals, Lot M8A9926), in a molar ratio of 0.75:2:1 (20% lipopeptide), 0.3:2:1 (10% lipopeptide) or 0.16:2:1 (5% lipopeptide), were dissolved in 2 ml of chloroform/methanol (3/1). The solvent was removed under reduced pressure using a rotary evaporator and the residue was dried in vacuo overnight. To this was added 5 ml of Dulbecco's phosphate buffer solution (free of Ca and Mg) (pH 7.4) and the mixture was treated with a bath-type sonicator for 30 minutes to give small unilamellar liposomes. The lipid not forming vesicles was removed by centrifugal ultrafiltration (1,500 × g, 15 min.; cut-off molecular weight 30,000, Amicon Centriprep-50).

For the liposomes thus obtained, the internal peptide content was determined with a commercial kit (Bio-Rad Protein Assay Kit) to confirm that the lipopeptide prepared as above had been incorporated in the liposomes.

### Example 10

### Peptide-modified liposomes

In 10 ml of chloroform were dissolved 6.7 mg of DDPE (dipalmitoyl L-α-phosphatidylethanolamine: Sigma), 45 mg of NHS-PEG-Mal (polyethylene glycol-α-N-β-maleimide hydroxysuccinimidyl propionate: Nichiyu Liposome Co.) and 980 µl of triethylamine, and the solution was stirred at room temperature. The reaction was monitored by TLC and after the reaction mixture was confirmed to be ninhydrin-negative, the solvent was distilled off. To the white slurry was added 2 ml of distilled water, and the mixture was treated with a bath-type sonicator to give miscelles. This solution was put in a cellophane tube of 2-fold capacity (cut-off molecular weight 12,000, size 20/30, Sanko Pure Chemicals Co.) and dialyzed against 1 L of distilled water for 3 days, with occasional change of the external fluid water to remove byproducts not forming miscelles. Further, the fraction in the neighborhood of the void was recovered by gel filtration (Sephadex G50 Fine, Amersham Pharmacia Biotech AB) and lyophilized to give the objective DPPE-PEG-Mal as a white powder giving a single band on the TLC.

The liposome (egg yolk lecithin/cholesterol = 2/1) prepared as in Example 9 was mixed with the above DPPE-PEG-Mal to prepare 5, 10, 20 and 100% DPPE-PEG-Mal-containing liposomes. Each of the above liposome solutions was adjusted to pH 8 with 1 N-NaOH/H₂O and the peptide of the invention, i.e. H-Gly-Cys-OH added to the C terminus of "A-1" peptide, was added in a predetermined final concentration. The mixture was stirred gently at room temperature for 6 hours and subjected to centrifugal ultrafiltration as in Example 9 to give a liposome solution. The peptide content of the liposome thus obtained was determined in the same manner as in Example 9.

In this manner, a modifier peptide consisting in an HA-binding peptide bound to the SH group of cysteine through the maleimide of DPPE-PEG-Mal was introduced onto the liposome to give a peptide-modified liposome.

### Example 11

### Evaluation of the inhibitory effect on the binding of hemagglutinin to GM3 cast film

The peptide expressed by clone "A-1" was synthesized by the procedure described in Example 8 and the inhibitory effect of this synthetic peptide on the binding of hemagglutinin to ganglioside GM3 was evaluated. Specifically, it was experimentally explored by ELISA whether the above synthetic peptide inhibits the binding of ganglioside GM3 to immobilized HA (H1N1, H3N2). As control, similar experiments were performed using the N-terminal 15-residue peptide of the phage coat protein pIII and sialyl-lactose in lieu of said synthetic peptide.

The results are presented in Fig. 7.

It is apparent from the above results that the A-1 peptide inhibits the binding of hemagglutinin to GM3 dose-dependently. In contrast, the peptide derived from the pIII protein showed no inhibitory effect. This finding suggested that the A-1 peptide binds to hemagglutinin with sequence specificity to thereby inhibit the binding of hemagglutinin to GM3. It was also suggested that sialyl-lactose competes with the sialic acid moiety of GM3 to thereby inhibit the binding between hemagglutinin and GM3.

### Example 12

### Evaluation of influenza virus infection inhibitory activity

The influenza virus infection inhibitory activity of the HA-binding peptide of the invention was evaluated.

As the influenza virus-infected cell, MDCK (Mardin-Darby canine kidney) cell was used. The influenza virus infection of this cell was verified by detecting LDH (lactate dehydrogenase) activity using a commercial kit (LDH-Cytotoxic Test, Wako) (J. Virol. Meth. 51, 185-192 (1995)). As the influenza virus, H1 (A/PR/8/34 (H1N1)) and H3 (A/Victoria/395 (H3N2)) influenza A viruses were respectively used.

Specifically, the infective titer of the influenza virus was determined as follows.

Thus, 3×10⁵ cells/ml of the MDCK cell grown in MEM/10% FBS were sown on a 96-well microtiter plate, 100 ml/well, and incubated under 5% CO₂ at 37°C overnight. After confirming that the cells in all wells had formed confluent monolayers, the wells were washed with serum-free MEM (MEM(-)) twice and a serial dilution of the virus solution (MEM(-)) was injected into the wells, 100 µl/well. The plate was incubated under 5% CO₂ at 37°C for 2 hours, after which the virus solution was discarded. The wells were washed with 2 portions of culture medium and incubated in the same medium for 20 hours. A 15 µl portion of the supernatant in each well was taken, diluted 4-fold with PBS to make 50 µl, and transferred to a fresh plate, in which 50 ml/well of a color developer (nitro blue tetrazolium, diaphorase and NDA+ in lithium lactate-PBS) was added. The plate was allowed to sit at room temperature for 20 minutes , after which 100 ml of a reaction stopper (0.5 M HCl in PBS) was added to the wells to stop the color reaction and the absorbance at 550 nm was measured with a microplate reader. The ID₅₀ value was calculated from the result obtained.

The experiment on the inhibition of influenza virus infection by the HA-binding peptide was carried out as follows. The test was performed with an amount of the virus corresponding to 50 times the ID₅₀ value found above (50 LDH ID₅₀). The results of influenza virus antiinfection experiments performed using the "C₁₈ A-1 lipopeptide" according to Example 9 and a liposome preparation containing 10% of this lipopeptide as the HA-binding peptide are presented in Figs. 8 and 9. Fig. 8 represents the result obtained with "C₁₈ A-1 lipopeptide" and Fig. 9 represents the result with the "C₁₈ A-1 lipopeptide"-modified liposome (10% peptide). On both diagrams, a) represents the antiinfective effect on influenza A virus H1 (A/PR/8/34 (H1N1)) and b) represents the antiinfective effect on H3 (A/Victoria/359 (H3N2)).

It will be apparent from Figs. 8 and 9 that "C₁₈ A-1 lipopeptide" effectively inhibits the influenza virus infection of cells and that this effect is potentiated by processing this peptide into a peptide-modified liposome. These findings suggested that the HA-binding peptide of the invention is of use as a prophylactic (antiinfective ) or therapeutic agent for influenza.

### INDUSTRIAL APPLICABILITY

In accordance with the present invention there is provided a peptide capable of binding specifically to influenza virus hemagglutinin.

The peptide of the invention binds specifically to the hemagglutinin associated with the first step of influenza virus infection to successfully inhibit binding of the virus to the host receptor and, as such, finds application as a prophylactic drug for influenza virus infection or a therapeutic drug for influenza. Particularly, the preferred peptide of the invention has the property to bind specifically to the receptor-binding pocket of hemagglutinin, the three-dimensional structure of which is well conserved among subtypes. Moreover, being a comparatively short peptide, the peptide of the invention is stable and because of the variegateness of its side chains, the peptide may undergo interactions with the binding sites of hemagglutinins. For this reason, the peptide of the invention is expected to find application as a broad-spectrum antiinfective or therapeutic drug for influenza, particularly for type A viruses in general, regardless of subtypes or over many subtypes.

Furthermore, for the very reason that it is a peptide, the peptide of the invention lends itself well to large-scale synthesis and modification as compared with the sugar chain analogs under development for use as antiinfluenza drugs, thus being useful for drug production and further drug development.

## Claims

1. An influenza virus hemagglutinin-binding peptide which is selected from:
(a) a peptide having any of the amino acid sequences defined under SEQ ID NO:1 through NO:11, and
(b) a peptide having an amino acid sequence derived from the above amino acid sequence (a) by the substitution, deletion or addition of one or a plurality of amino acids and capable of binding to influenza virus hemagglutinin.

2. An influenza virus hemagglutinin-binding peptide having the amino acid sequence defined under SEQ ID NO:1 or NO:6.

3. An influenza virus hemagglutinin-binding peptide having any of the amino acid sequences defined under SEQ ID NO:7 through NO:11.

4. The influenza virus
hemagglutinin-binding peptide according to any of Claims 1 through 3 wherein the influenza virus is a type A virus.

5. The influenza virus
hemagglutinin-binding peptide according to any of Claims 1 through 3 wherein the influenza virus is a subtype H1 virus.

6. The influenza virus
hemagglutinin-binding peptide according to any of Claims 1 through 3 wherein the influenza virus is a subtype H3 virus.

7. The influenza virus
hemagglutinin-binding peptide according to Claim 1 which has been alkylated or lipidated.

8. A liposome containing the influenza virus hemagglutinin-binding peptide of Claim 7.

9. An influenza virus hemagglutinin binding inhibitor comprising the influenza virus
hemagglutinin-binding peptide of Claim 1 as an active ingredient.

10. An influenza virus hemagglutinin binding inhibitor comprising the influenza virus
hemagglutinin-binding peptide of Claim 7 as an active ingredient.

11. An influenza virus hemagglutinin binding inhibitor comprising the liposome of Claim 8 as an active ingredient.

12. An influenza virus hemagglutinin binding inhibitor comprising an influenza virus
hemagglutinin-binding peptide having the amino acid sequence defined under SEQ ID NO:1 or NO:6 as an active ingredient.

13. An influenza virus hemagglutinin binding inhibitor comprising an influenza virus
hemagglutinin-binding peptide having any of the amino acid sequences defined under SEQ ID NO:7 through NO:11 as an active ingredient.

14. A pharmaceutical composition comprising the influenza virus hemagglutinin-binding peptide of Claim 1 as an active ingredient in combination with a pharmaceutically acceptable carrier.

15. A pharmaceutical composition comprising the influenza virus hemagglutinin-binding peptide of Claim 7 as an active ingredient in combination with a pharmaceutically acceptable carrier.

16. A pharmaceutical composition comprising the liposome of Claim 8 as an active ingredient in combination with a pharmaceutically acceptable carrier.

17. A pharmaceutical composition comprising an influenza virus hemagglutinin-binding peptide having the amino acid sequence defined under SEQ ID NO:1 or NO:6 as an active ingredient in combination with a pharmaceutically acceptable carrier.

18. A pharmaceutical composition comprising an influenza virus hemagglutinin-binding peptide having any of the amino acid sequences defined under SEQ ID NO:7 through NO:11 as an active ingredient in combination with a pharmaceutically acceptable carrier.

19. A pharmaceutical composition according to Claim 14 wherein the influenza virus of interest is a type A virus.

20. A pharmaceutical composition according to any of Claims 14 through 16 which is used as an antiinfluenza drug.

21. A method for prophylaxis or therapy of influenza which comprises administering an effective amount of the influenza virus hemagglutinin-binding peptide according to Claim 1 to a recipient individual.

22. A method for prophylaxis or therapy of influenza which comprises administering an
effective amount of the influenza virus
hemagglutinin-binding peptide according to Claim 7 to a recipient individual.

23. A method for prophylaxis or therapy of influenza which comprises administering an effective amount of the liposome according to Claim 8 to a recipient individual.

24. A method for prophylaxis or therapy of influenza which comprises administering the pharmaceutical composition according to any of Claims 14 through 16 to a recipient individual.

25. Use of the influenza virus
hemagglutinin-binding peptide according to Claim 1 as an antiinfluenza drug.

26. Use of the influenza virus
hemagglutinin-binding peptide according to Claim 7 as an antiinfluenza drug.

27. Use of the liposome according to Claim 8 as an antiinfluenza drug.

28. Use of the influenza virus
hemagglutinin-binding peptide according to Claim 1 for the production of a pharmaceutical composition to be used as an antiinfluenza drug.

29. Use of the influenza virus
hemagglutinin-binding peptide according to Claim 7 for the production of a pharmaceutical composition to be used as an antiinfluenza drug.

30. Use of the liposome according to Claim 8 for the production of a pharmaceutical composition to be used as an antiinfluenza drug.


SEQUENCE LISTING

<110> Otsuka Pharmaceutical Co. Ltd.

<120> Affinity peptides to Hemagglutinin of Influenza
<130> P00-03

<140>
<141>
<150> JP H11-91962
<151> 1999-03-31
<160> 11
<170>

<210> 1
<211> 15
<212> PRT
<213> phage library
<220>
<400> 1
Gly Tyr Cys Ser Trp Phe Pro Ala Trp Ser Gly Cys Ser Ala Gly
 1               5                   10                  15

<210> 2
<211> 14
<212> PRT
<213> phage library
<220>
<400> 2
Gln Gly Trp Arg Ala Leu Leu Phe Arg Ser Pro Val Asn Arg
 1               5                   10

<210> 3
<211> 15
<212> PRT
<213> phage library
<220>
<400> 3

Gly Trp Arg Val Tyr Leu Ser Asn Leu Trp Asp Glu Arg Arg Gly
1               5                   10                  15

<210> 4
<211> 15
<212> PRT
<213> phage library
<220>
<400> 4
Ser Arg Leu Val Gly Tyr Cys Thr Arg Ser Pro Ala Val Cys Arg
1               5                   10                  15

<210> 5
<211> 15
<212> PRT
<213> phage library
<220>
<400> 5
Phe His Cys Val Ser Asp Gly Phe Phe Ser Ser Arg Cys Tyr Arg
1               5                   10                  15

<210> 6
<211> 15
<212> PRT
<213> phage library
<220>
<400> 6
Gly Tyr Gly Ser Trp Phe Pro Ala Leu Ser Gly Cys Ser Thr Gly
1               5                   10                  15

<210> 7
<211> 15
<212> PRT
<213> phage library
<220>
<400> 7
Ala Arg Leu Ser Pro Thr Met Val His Pro Asn Gly Ala Gln Pro
1               5                   10                  15

<210> 8
<211> 15
<212> PRT
<213> phage library
<220>
<400> 8
Gly Arg Val Pro Val Phe Gly Leu Ser Pro Leu Phe Lys Val Glu
1               5                   10                  15

<210> 9
<211> 15
<212> PRT
<213> phage library
<220>
<400> 9
Gly Arg Pro Pro Asp Ser Val Phe Arg Ser Arg Gly Trp Leu Ser
1               5                   10                  15

<210> 10
<211> 15
<212> PRT
<213> phage library
<220>
<400> 10
Ile Asp Ile Ala Phe Ser Ser Leu Ala Leu Ala Asp Ile Ser Arg
1               5                   10                  15

<210> 11
<211> 15
<212> PRT
<213> phage library
<220>
<400> 11
Glu Pro Tyr Gly Phe Ile Ala Phe Ser Arg Ala Ala His Ser Pro
1               5                   10                  15